# EUROPÄISCHE PATENTSCHRIFT

(11) **EP 1 279 658 B1**
(45) Veröffentlichungstag und Bekanntmachung des Hinweises auf die Patenterteilung: **04.02.2009**
(21) Anmeldenummer: 02012800.5
(22) Anmeldetag: 10.06.2002
(51) Int. Cl.: C07C 45/50, C07C 29/156

(54) **Verfahren zur Hydroformylierung von höheren Olefinen mit Kobaltverbindungen als Katalysator**
Process for the hydroformylation of higher olefins in presence of cobalt compounds as catalyst
Procédé pour l'hydroformylation d'oléfines supérieures en présence de composés de cobalt comme catalyseur

(30) Priorität: 24.07.2001 DE 10135906
(43) Veröffentlichungstag der Anmeldung: 29.01.2003
(73) Patentinhaber: Evonik Oxeno GmbH, 45772 Marl (DE)
(72) Erfinder: Scholz, Bernhard, Dr., 45768 Marl (DE); Tötsch, Walter, Dr., 45770 Marl (DE); Drees, Stefan, 48249 Dülmen (DE); Kaizik, Alfred, Dr., 45772 Marl (DE); Trocha, Martin, Dr., 45136 Essen (DE); Büschken, Wilfried, Dr., 45721 Haltern (DE)
(74) Vertreter: Hirsch, Hans-Ludwig

(56) Entgegenhaltungen:
- EP-A- 0 850 905
- DE-A- 19 939 491
- DE-B- 1 106 307
- GB-A- 875 565

## Beschreibung

Die Erfindung betrifft ein Verfahren zur Hydroformylierung von höheren Olefinen in Gegenwart von unmodifizierten Kobaltcarbonylkomplexen durch Aufrechterhaltung einer wässrigen Sumpfphase im Hydroformylierungsreaktor.

Höhere Alkohole, insbesondere solche mit 6 bis 25 Kohlenstoffatomen, können bekanntlich durch katalytische Hydroformylierung (Oxo-Reaktion) der um ein Kohlenstoffatom kürzeren Olefine und anschließende katalytische Hydrierung der Aldehyd- und Alkohol-haltigen Reaktionsgemische hergestellt werden. Sie werden vorwiegend für die Herstellung von Weichmachern und Detergentien verwendet. Es ist weiterhin aber auch möglich, durch Destillation der Hydroformylierungsgemische Aldehyde abzutrennen. Diese können beispielsweise zur Herstellung von Carbonsäuren genutzt werden.

Die Art des Katalysatorsystems und die optimalen Reaktionsbedingungen für die Hydroformylierung sind von der Reaktivität des eingesetzten Olefins abhängig. Die Abhängigkeit der Reaktivität der Olefine von ihrer Struktur ist z. B. von J. Falbe, New Syntheses with Carbon Monoxide, Springer-Verlag, Berlin, Heidelberg, New York, 1980, Seite 95 ff., beschrieben. Die unterschiedliche Reaktivität speziell der isomeren Octene ist ebenfalls bekannt (B. L. Haymore, A. van Hasselt, R. Beck, Annals of the New York Acad. Sci., 415 (1983), Seiten 159 - 175).

Technische Olefingemische, die als Edukte für die Oxo-Synthese verwendet werden, enthalten Olefinisomere der verschiedensten Strukturen mit unterschiedlichen Verzweigungsgraden, unterschiedlicher Lage der Doppelbindung im Molekül und gegebenenfalls auch unterschiedlichen Kohlenstoffzahlen. Dies gilt insbesondere für Olefingemische, die durch Di- oder Trimerisierung sowie weitergehende Oligomerisierung von C₂ - C₅-Olefinen oder anderen leicht zugänglichen höheren Olefinen beziehungsweise durch Cooligomerisierung der genannten Olefine entstanden sind. Als Beispiele für typische isomere Olefingemische, die durch Rhodium-katalysierte oder vorzugsweise durch Kobalt-katalysierte Hydroformylierung zu den entsprechenden Aldehydund Alkohol-Gemischen umgesetzt werden können, seien Tri- und Tetrapropene sowie Di- , Tri- und Tetrabutene genannt.

Sind Alkohole mit möglichst geringem Verzweigungsgrad als Hydroformylierungsprodukt angestrebt, wird die Hydroformylierung vorteilhaft mit unmodifizierten Kobaltkatalysatoren durchgeführt Im Vergleich zu Rhodiumkatalysatoren werden mit Kobaltkatalysatoren, ausgehend von einem gleichen Olefingemisch, höhere Ausbeuten an den besonders wertvollen geradkettigen Oxo-Produkten erhalten.

Die Hydroformylierung von Olefinen mit unmodifizierten Kobaltkatalysatoren kann, abgesehen von der Katalysatoraufarbeitung, ein- oder mehrstufig durchgeführt werden.

Die bekannten mehrstufigen Herstellungsverfahren von Oxo-Aldehyden in Gegenwart von unmodifizierten Kobaltkatalysatoren weisen eine Reihe von technischen Nachteilen auf. So sind für die Herstellung des für die Hydroformylierung benötigten Kobaltkatalysators zwei technisch aufwendige Verfahrensstufen, Vorcarbonylierung und Katalysatorextraktion, erforderlich. Bedingt durch die in den beiden Verfahrensstufen ablaufenden Stoffübergangsvorgänge, Gas/Flüssigkeit-Stoffübergang bei der Vorcarbonylierung und Flüssigkeit/Flüssigkeit-Stoffübergang bei der Katalysatorextraktion, sind zwei voneinander getrennte druckfeste Apparate, wie zum Beispiel Rührkessel oder Füllkörperkolonnen, erforderlich. Die eigentliche Hydroformylierung findet anschließend wiederum in einem separaten Druckreaktor statt.

Die deutsche Patentanmeldung DE 196 54 340 beschreibt ein Verfahren, in dem Vorcarbonylierung, Katalysatorextraktion und Olefin-Hydroformylierung in einem Reaktor durchgeführt werden. Dieser Prozess hat gegenüber den bekannten mehrstufigen Verfahren die Vorteile einer geringeren Kapitalanlage und geringerer Betriebskosten. Nachteilig ist jedoch, dass die Prozessführung des einstufigen Prozesses ziemlich schwierig ist, da die Verfahrensteilschritte wie Katalysatorbildung, Extraktion des Katalysators in die organische Phase und Hydroformylierung simultan erfolgen. Im Reaktor liegt im unteren Teil eine wässrige Kobaltsalzlösung vor, in der der Katalysator gebildet wird. Die Hydroformylierung geschieht hauptsächlich in der homogenen organischen Phase. Mit dem den Reaktor verlassenden Hydroformylierungsgemisch und dem abgezogenen Synthesegas werden kontinuierlich Wasser und Kobaltverbindungen aus dem Reaktor ausgetragen, so dass Kobaltverbindungen und Wasser ständig nachdosiert werden müssen.

In DE 199 39 491 A1 wird ein Hydroformylierungsverfahren beschrieben, bei dem die Bildung des Kobaltkatalysators, die Extraktion des Kobaltkatalysators in die organische Phase und die Hydroformylierung der Olefine in einem Schritt erfolgen, wobei die Phasen innig in Kontakt gebracht werden. Der unvermeidliche Wasserentzug wird durch periodische oder kontinuierliche Wasserergänzung kompensiert.

In GB-A-875565 wird ebenfalls ein einstufiges Hydroformylierungsverfahren beschrieben, bei dem die beiden flüssigen Phasen getrennt gehalten werden und die Durchmischung nur an der Phasengrenze erfolgt. Eine Erneuerung der Wasserphase kann kontinuierlich oder in Abständen erfolgen, um deren Volumen und Zusammensetzung im Wesentlichen konstant zu halten.

Obwohl sich das einstufige Verfahren im Großen und Ganzen bewährt, kommt es im Betrieb zu Schwankungen des Umsatzes und der Selektivität sowie zur Störung des kontinuierlichen Betriebs durch Ausfällungen von Kobaltverbindungen und/oder metallischem Kobalt.

Es bestand daher die Aufgabe, die Prozessführung des Einstufenprozesses derart zu verbessern, dass die genannten Beeinträchtigungen des kontinuierlichen Betriebes vermieden und die Zielprodukte in hoher Ausbeute und in hoher Selektivität erhalten werden.

Es wurde nun gefunden, dass ein Einstufenverfahren zur Hydroformylierung von Olefinen, das die Zielprodukte in hohen Ausbeuten liefert, vorteilhaft betrieben werden kann, wenn durch geeignete technische Maßnahmen die wässrige Sumpfphase mit der organischen Phase durchmischt wird, wenn die Kobaltkonzentration (berechnet als metallisches Kobalt) in der wässrigen. Sumpfphase auf Werte von 0,4 bis 1,7 Massen-% eingestellt wird und wenn eine wässrige Sumpfphase mit konstantem Stand bei stationärem Betrieb im Reaktor aufrechterhalten wird.

Gegenstand der vorliegenden Erfindung ist daher ein Verfahren zur Hydroformylierung von Olefinen mit 5 bis 24, insbesondere 5 bis 12, bevorzugt 5 bis 10 und besonders bevorzugt mit 8 oder 9 Kohlenstoffatomen zu den entsprechenden Aldehyden und/oder Alkoholen mit 6 bis 25, insbesondere 6 bis 13, bevorzugt 6 bis 11 und besonders bevorzugt 9 oder 10 Kohlenstoffatomen in Gegenwart von unmodifizierten Kobaltkatalysatoren, das in einem Einstufenprozess bei Temperaturen von 100 °C bis 220 °C, insbesondere 120 bis 160 °C und Drücken von 100 bar bis 400 bar, vorzugsweise 200 bis 280 bar bei Vorliegen einer wässrigen und organischen Phase im Reaktor durchgeführt wird, das dadurch gekennzeichnet ist, dass die wässrige Sumpfphase im Reaktor mit der organischen Phase durchmischt wird, die Konzentration an Kobaltverbindungen (berechnet als metallisches Kobalt) in der wässrigen Sumpfphase im Bereich von 0,4 bis 1,7 Massen-% liegt und der Stand der wässrigen Sumpfphase im Reaktor im stationären Zustand konstant gehalten wird, wobei die Olefine mit einer wässrigen Kobaltsalzlösung und Synthesegas mittels einer Mischdüse in den Reaktor gebracht werden und die Phasengrenze zwischen wässriger und organischer Phase 0 bis 1 m unter oder über der Austrittsöffnung der Mischdüse liegt.

Die Durchführung der Hydroformylierung und die Abtrennung des Kobaltkatalysators vom Hydroformylierungsaustrag kann zum Beispiel wie in der deutschen Patentanmeldung DE 196 54 340 A1 beschrieben, erfolgen. Es handelt sich hier um einen 1-Stufen-Prozess, bei dem die Olefine mit einer wässrigen Kobaltsalzlösung und Synthesegas, besonders bevorzugt mittels einer Mischdüse, in den Reaktor eingebracht werden. Der Reaktor ist bevorzugt ein kaskadierter Blasensäulenreaktor. Der Reaktoraustrag wird anschließend oxidativ in Gegenwart einer wässrigen Lösung von Carbonsäuren, vorwiegend von Ameisensäure, behandelt. Nach Phasentrennung wird ein Teil der wässrigen, die Kobaltsalze enthaltenden Phase in den 1-Stufen-Prozess zurückgeführt und die organische Phase zu den Produkten (Aldehyd) weiter aufgearbeitet oder zu den entsprechenden Alkoholen hydriert. Die in den Hydroformylierungsreaktor zurückgeführte wässrige Lösung enthält praktisch so viel Kobalt, wie mit dem Hydroformylierungsgemisch ausgetragen wird.

Im erfindungsgemäßen Verfahren wird der Stand der wässrigen Sumpfphase im Hydroformylierungsreaktor konstant oder nahezu konstant gehalten. Dies bedeutet, dass während eines stationären Betriebes (konstante Betriebsbedingungen) die Phasengrenze zwischen unterer wässriger Phase, in der ein Teil der organischen Phase dispergiert ist, und der oberen Phase, in der ein Teil der wässrigen Phase dispergiert ist, einen Stand annimmt, dessen Höhe maximal um ± 5 % um einen Mittelwert schwankt. Dieser Mittelwert der Höhe der Phasengrenze kann im erfindungsgemäßen Verfahren unterhalb oder oberhalb oder in der Höhe der Austrittsöffnung der Mischdüse, durch die die Edukte in den Reaktor eingebracht werden, liegen. Die Phasengrenze kann sich dabei bei einer technischen Ausführungsform beispielsweise 0 bis 1 m, insbesondere 0 bis 0,5 m, ganz besonders bevorzugt 0 bis 0,2 m über oder unter der Austrittsöffnung der Mischdüse befinden.

Es ist wichtig, die Trennschicht im Reaktor konstant zu halten, da ansonsten Schwankungen der Kobaltkonzentration in der wässrigen Phase unvermeidbar sind, wodurch Betriebsstörungen verursacht werden können. Aber auch die absolute Höhe der Trennschicht hat Einfluss auf das Reaktionsverhalten. Liegt die Trennschicht deutlich oberhalb der Mischdüse, kommt es zu einer Verringerung des Umsatzes, weil die Ejektorwirkung der Mischdüse nicht mehr voll wirksam ist. Eine zu niedrige Trennschicht führt zu lokalen Temperaturspitzen, welche eine Zersetzung des Katalysators verursachen können. Die optimale Höhe der Trennschicht zur Erreichung einer maximalen Ausbeute und/oder Selektivität hängt somit von den spezifischen Konzentrationen im Reaktor ab, beispielsweise von der Kobaltkonzentration in der wässrigen Phase, sowie von den anderen Prozessparametern; die Trennschicht muss daher ständig an die vorliegenden Betriebsbedingungen angepasst werden.

Die wässrige Phase im unteren Teil des Reaktors beträgt 0,5 bis 20 %, insbesondere 1 bis 10 % des flüssigen Reaktorinhaltes.

Der Wasseraustrag aus dem Reaktor durch das flüssige Hydroformylierungsgemisch und durch das überschüssige Synthesegas ist keine konstante Größe. Dieser hängt von den Betriebsparametern, wie Druck, Temperatur, Katalysatorkonzentration und Verweilzeit, ganz besonders jedoch von der Zusammensetzung des Hydroformylierungsgemisches ab, die das Wasserlösevermögen und somit den Wasseraustrag mitbestimmt. Insbesondere die durch Hydrierung der Aldehyde entstandenen Alkohole erhöhen die Wasserlöslichkeit. Darüber hinaus ändert sich der Wasseraustrag bei Anfahrvorgängen oder Lastwechseln.

Nach dem erfindungsgemäßen Verfahren kann zur Aufrechterhaltung des Standes der wässrigen Phase Frischwasser und/oder Wasser, abgetrennt an einer anderen Stelle des Verfahrens, eingespeist werden. Die zurückgeführten Wasserströme können Edukte, Produkte und Kobaltverbindungen enthalten.

Das Wasser kann direkt in den Reaktorsumpf eingebracht werden. Eine andere Möglichkeit besteht darin, Wasser zusammen mit Olefin und/oder Synthesegas einzuspeisen. Weiterhin kann Wasser beziehungsweise eine wässrige Lösung in eine gegebenenfalls vorhandene Umlaufleitung für die wässrige Reaktorphase gepumpt werden.

Auch der Austrag von Kobaltverbindungen ist zeitlich nicht konstant, sondern von den Betriebsparametern abhängig.

Zur Einstellung der Kobaltkonzentration in der wässrigen Sumpfphase des Reaktors werden im erfindungsgemäßen Verfahren Kobaltverbindungen zudosiert. Die Kobaltverbindungen werden als Lösung, beispielsweise gelöst in Produkt, Edukt oder Wasser, eingespeist. Bevorzugt werden wässrige Lösungen von Kobaltsalzen von Carbonsäuren, wie beispielsweise Kobaltformiat oder Kobaltacetat, eingesetzt. Es können auch Lösungen, die mehr als eine Kobaltverbindung enthalten, eingesetzt werden. Eine besonders bevorzugte Kobaltsalzlösung ist diejenige, die im Prozess selbst bei der Entkobaltung des Hydroformylierungsaustrags anfällt. Diese Lösung, die auch Ameisensäure enthält, kann direkt oder nach Aufkonzentrierung oder nach Reduzierung des Ameisensäuregehalts, zum Beispiel nach der deutschen Patentanmeldung DE 100 09 207.1 eingesetzt werden.

Auf jeden Fall wird so viel Kobalt und Wasser nachdosiert, dass der Stand der wässrigen Sumpfphase möglichst konstant gehalten wird und die Konzentration der Kobaltverbindungen (gerechnet als metallisches Kobalt) in der wässrigen Sumpfphase des Reaktors im Bereich von 0,4 bis 1,7 Massen-%, insbesondere im Bereich von 0,7 bis 1,3 Massen-% aufrechterhalten wird. Niedrigere Kobaltkonzentrationen sind nicht sinnvoll, da dann die Reaktionsgeschwindigkeit zu langsam wird. Höhere Kobaltkonzentrationen sind ebenso zu vermeiden, da sie die Abscheidung von Kobaltverbindungen oder metallischem Kobalt begünstigen. Dadurch können Verstopfungen auftreten und die Funktion von Mess- und Regeleinrichtungen beeinträchtig werden, was zu Betriebsstörungen führen kann. Die Kobaltkonzentration wird überwacht, zweckmäßig durch laufende Analytik (On-line-Analytik).

Um eine hohe Reaktionsgeschwindigkeit zu erhalten, ist es zweckmäßig, die wässrige Sumpfphase mit der organischen Phase und Synthesegas sowie die wässrige Phase zu vermischen. Durch die intensive Vermischung werden Konzentrationsgradienten der Reaktionspartner vermieden. Darüber hinaus begünstigt die Vermischung der wässrigen Sumpfphase mit der organischen Phase den Übergang des gebildeten Katalysators in die organische Phase, in der die Hydroformylierung hauptsächlich abläuft.

Die Vermischung der Reaktionskomponenten (Olefin, Synthesegas, wässrige Kobaltsalzlösung) mit sich selbst und/oder Hydroformylierungsgemisch sowie die Vermischung der beiden flüssigen Phasen im Reaktor kann mit Hilfe geeigneter technischer Einrichtungen erfolgen.

Olefin, Synthesegas und wässrige Kobaltsalzlösung können getrennt, zweckmäßig durch Düsen, in den Reaktor eingeleitet werden. Es können auch zwei Komponenten zusammen durch eine oder mehrere Mischdüsen und die dritte Komponente getrennt in den Reaktor eingespeist werden. Zweckmäßig ist es jedoch, alle drei Komponenten zusammen durch eine oder mehrere Mischdüsen dem Reaktor zuzuführen.

Die wässrige Sumpfphase kann mit Hilfe einer Pumpe, die in einer Umlaufleitung installiert ist, umgewälzt werden. Eine Durchmischung der wässrigen Phase und Vermischung der wässrigen Phase mit der organischen Phase und Synthesegas kann auch dadurch erreicht werden, dass ein Teil der wässrigen Phase aus dem Reaktor der Mischdüse für die Einsatzstoffe zugeleitet wird. Dies kann mit Hilfe einer Pumpe geschehen. Eine andere Möglichkeit besteht darin, einen Teil der wässrigen Sumpfphase aus dem Reaktor von der Stoffströmung in der Mischdüse ansaugen zu lassen.

Die Ejektorwirkung von Mischdüsen wird durch den Impuls des austretenden Gases und der austretenden Flüssigkeit beeinflusst. Hohe Flüssigkeitsgeschwindigkeiten von 3 bis 300 m/s, besonders 10 bis 100 m/s, ganz besonders von 15 bis 70 m/s am Ort beziehungsweise an den Orten der Einmischung sind vorteilhaft.

Die Edukte für das erfindungsgemäße Verfahren sind Olefine oder Gemische von Olefinen mit 5 bis 24, insbesondere 5 bis 12, vorzugsweise 5 bis 10 und besonders bevorzugt 8 oder 9 Kohlenstoffatomen mit end- und/oder innenständigen C-C-Doppelbindungen. Besonders bevorzugte Edukte sind Gemische isomerer Octene und Nonene, das heißt Oligomere von niedrigen Olefinen, wie n-Butenen, Isobuten oder Propen. Andere ebenfalls gut geeignete Edukte sind Dimere von C₅-Olefinen Für die Oligomerisierung von Butenen zu im Wesentlichen C₈-Olefinen enthaltenden Gemischen gibt es dabei im Prinzip drei Verfahrensvarianten. Lange bekannt ist die Oligomerisierung an sauren Katalysatoren, wobei technisch zum Beispiel Zeolithe oder Phosphorsäure auf Trägern eingesetzt werden. Hierbei werden Isomerengemische von verzweigten Olefinen erhalten, die im Wesentlichen Dimethylhexene darstellen (WO 92/13818). Ein ebenfalls weltweit ausgeübtes Verfahren ist die Oligomerisierung mit löslichen Nickel-Komplexen, bekannt als DIMERSOL-Verfahren (B. Cornils, W. A. Herrmann, Applied Homogeneous Catalysis with Organicmetallic Compounds; Vol. 1&2, S. 258 - 264, VCH, Weinheim, New York 1996). Die dritte Verfahrensvariante ist die Oligomerisierung an Nickel-Festbett-Katalysatoren; eines dieser Verfahren hat Eingang in die Literatur als OCTOL-Prozess (Hydrocarbon Process., Int. Ed. (1986) 65 (2. Sect.1) Seite 31 - 33) gefunden. Vorzugsweise werden für das erfindungsgemäße Verfahren Olefine beziehungsweise Olefingemische eingesetzt, die durch Oligomerisierung an Nickel-Festbettkatalysatoren hergestellt wurden.

Die Hydroformylierungsgemische können zur Herstellung von Aldehyden verwendet werden. Aus den Hydroformylierungsgemischen können aber auch durch Hydrierung die entsprechenden Alkohole hergestellt werden. Ein wichtiges Einsatzgebiet der so herstellbaren Alkohole ist die Verwendung zur Herstellung von Weichmachern wie zum Beispiel Phthalsäureestern.

## Patentansprüche

1. Verfahren zur Hydroformylierung von Olefinen mit 5 bis 24 Kohlenstoffatomen zu den entsprechenden Aldehyden und/oder Alkoholen mit 6 bis 25 Kohlenstoffatomen in Gegenwart von unmodifizierten Kobaltkatalysatoren in einem Einstufenprozess bei Temperaturen von 100 °C bis 220 °C und Drücken von 100 bar bis 400 bar bei Vorliegen einer wässrigen und organischen Phase im Reaktor,
**dadurch gekennzeichnet, dass** die wässrige Sumpfphase im Reaktor mit der organischen Phase durchmischt wird, die Konzentration an Kobaltverbindungen (berechnet als metallisches Kobalt) in der wässrigen Sumpfphase im Bereich von 0,4 bis 1,7 Massen-% liegt und der Stand der wässrigen Sumpfphase im Reaktor im stationären Zustand konstant gehalten wird, wobei die Olefine mit einer wässrigen Kobaltsalzlösung und Synthesegas mittels einer Mischdüse in den Reaktor gebracht werden und die Phasengrenze zwischen wässriger und organischer Phase 0 bis 1 m unter oder über der Austrittsöffnung der Mischdüse liegt.

2. Verfahren nach Anspruch 1,
**dadurch gekennzeichnet, dass** die Konzentration an Kobaltverbindungen (berechnet als metallisches Kobalt) in der wässrigen Phase im Bereich von 0,7 bis 1,3 Massen- % liegt.

3. Verfahren nach Anspruch 1 oder 2,
**dadurch gekennzeichnet, dass** die wässrige Sumpfphase im Reaktor 0,5 bis 20 % des flüssigen Reaktorinhalts beträgt.

4. Verfahren nach einem der Ansprüche 1 bis 3,
**dadurch gekennzeichnet, dass** die wässrige Sumpfphase im Reaktor 1 bis 10 % des flüssigen Reaktorinhaltes beträgt.

5. Verfahren nach einem der Ansprüche 1 bis 4,
**dadurch gekennzeichnet, dass** die wässrige Sumpfphase durch die Ejektorwirkung der Mischdüse mit der organischen Phase durchmischt wird.

6. Verfahren nach Anspruch 5,
**dadurch gekennzeichnet, dass** die Flüssigkeitsgeschwindigkeit am Austritt der Mischdüse 3 bis 300 m/s beträgt.

7. Verfahren nach Anspruch 6,
**dadurch gekennzeichnet,**
**dass** die Flüssigkeitsgeschwindigkeit am Austritt der Mischdüse 10 bis 100 m/s beträgt.

8. Verfahren nach einem der Ansprüche 1 bis 7,
**dadurch gekennzeichnet,**
**dass** die wässrige Phase mit Hilfe einer Umwälzpumpe mit der organischen Phase durchmischt wird.

9. Verfahren nach einem der Ansprüche 1 bis 8,
**dadurch gekennzeichnet,**
**dass** Olefine mit 5 bis 12 Kohlenstoffatomen eingesetzt werden.

10. Verfahren nach einem der Ansprüche 1 bis 8,
**dadurch gekennzeichnet,**
**dass** Olefine mit 5 bis 10 Kohlenstoffatomen eingesetzt werden.

11. Verfahren nach einem der Ansprüche 1 bis 8,
**dadurch gekennzeichnet,**
**dass** Olefine oder Olefingemische mit 8 oder 9 Kohlenstoffatomen eingesetzt werden.

12. Verfahren nach Anspruch 11,
**dadurch gekennzeichnet,**
**dass** C₈-Olefingemische, die durch Oligomerisierung am Nickel-Festbettkatalysator hergestellt wurden, eingesetzt werden.

## Claims

1. Process for the hydroformylation of olefins having from 5 to 24 carbon atoms to form the corresponding aldehydes and/or alcohols having from 6 to 25 carbon atoms in the presence of unmodified cobalt catalysts in a single-stage process at temperatures of from 100°C to 220°C and pressures of from 100 bar to 400 bar, with an aqueous and organic phase being present in the reactor, **characterized in that** the aqueous bottom phase in the reactor is mixed with the organic phase, the concentration of cobalt compounds (calculated as metallic cobalt) in the aqueous bottom phase is in the range from 0.4 to 1.7% by mass and the level of the aqueous bottom phase in the reactor is kept constant during steady-state operation, where the olefins are introduced into the reactor together with an aqueous cobalt salt solution and synthesis gas by means of a mixing nozzle and the phase boundary between the aqueous phase and the organic phase is from 0 to 1 m below or above the exit orifice of the mixing nozzle.

2. Process according to Claim 1, **characterized in that** the concentration of cobalt compounds (calculated as metallic cobalt) in the aqueous phase is in the range from 0.7 to 1.3% by mass.

3. Process according to Claim 1 or 2, **characterized in that** the aqueous bottom phase in the reactor makes up from 0.5 to 20% of the liquid contents of the reactor.

4. Process according to any of Claims 1 to 3, **characterized in that** the aqueous bottom phase in the reactor makes up from 1 to 10% of the liquid contents of the reactor.

5. Process according to any of Claims 1 to 4, **characterized in that** the aqueous phase is mixed with the organic phase by means of the ejector action of the mixing nozzle.

6. Process according to Claim 5, **characterized in that** the liquid velocity at the outlet of the mixing nozzle is from 3 to 300 m/s.

7. Process according to Claim 6, **characterized in that** the liquid velocity at the outlet of the mixing nozzle is from 10 to 100 m/s.

8. Process according to any of Claims 1 to 7, **characterized in that** the aqueous phase is mixed with the organic phase by means of a circulation pump.

9. Process according to any of Claims 1 to 8, **characterized in that** olefins having from 5 to 12 carbon atoms are used.

10. Process according to any of Claims 1 to 8, **characterized in that** olefins having from 5 to 10 carbon atoms are used.

11. Process according to any of Claims 1 to 8, **characterized in that** olefins or olefin mixtures having 8 or 9 carbon atoms are used.

12. Process according to Claim 11, **characterized in that** C₈-olefin mixtures which have been prepared by oligomerization over a fixed-bed nickel catalyst are used.

## Revendications

1. Procédé d'hydroformylation d'oléfines ayant 5 à 24 atomes de carbone en aldéhydes et/ou alcools correspondants ayant 6 à 25 atomes de carbone en présence de catalyseurs de cobalt non modifiés par un procédé en une étape à des températures de 100 °C à 220 °C et des pressions de 100 bars à 400 bars en présence d'une phase aqueuse et d'une phase organique dans le réacteur,
**caractérisé en ce que** la phase de fond aqueuse dans le réacteur est mélangée avec la phase organique, la concentration en composés de cobalt (calculée en tant que cobalt métallique) dans la phase de fond aqueuse est de 0,4 à 1,7 % en masse et le niveau de la phase de fond aqueuse dans le réacteur à l'état stationnaire est maintenu constant, les oléfines étant introduites dans le réacteur avec une solution aqueuse de sel de cobalt et du gaz de synthèse par une buse de mélange et la séparation de phases entre la phase aqueuse et la phase organique se trouvant 0 à 1 m au-dessous ou au-dessus de l'orifice de sortie de la buse de mélange.

2. Procédé selon la revendication 1,
**caractérisé en ce que** la concentration en composés de cobalt (calculée en tant que cobalt métallique) dans la phase aqueuse est de 0,7 à 1,3 % en masse.

3. Procédé selon la revendication 1 ou 2,
**caractérisé en ce que** la phase de fond aqueuse dans le réacteur représente 0,5 à 20 % du contenu liquide du réacteur.

4. Procédé selon l'une quelconque des revendications 1 à 3,
**caractérisé en ce que** la phase de fond aqueuse dans le réacteur représente 1 à 10 % du contenu liquide du réacteur.

5. Procédé selon l'une quelconque des revendications 1 à 4,
**caractérisé en ce que** la phase de fond aqueuse est mélangée avec la phase organique par l'action d'éjection de la buse de mélange.

6. Procédé selon la revendication 5,
**caractérisé en ce que** la vitesse du liquide à la sortie de la buse de mélange est de 3 à 300 m/s.

7. Procédé selon la revendication 6,
**caractérisé en ce que**
la vitesse du liquide à la sortie de la buse de mélange est de 10 à 100 m/s.

8. Procédé selon l'une quelconque des revendications 1 à 7,
**caractérisé en ce que**
la phase aqueuse est mélangée avec la phase organique à l'aide d'une pompe de circulation.

9. Procédé selon l'une quelconque des revendications 1 à 8,
**caractérisé en ce que**
des oléfines ayant 5 à 12 atomes de carbone sont utilisées.

10. Procédé selon l'une quelconque des revendications 1 à 8,
**caractérisé en ce que**
des oléfines ayant 5 à 10 atomes de carbone sont utilisées.

11. Procédé selon l'une quelconque des revendications 1 à 8,
**caractérisé en ce que**
des oléfines ou des mélanges d'oléfines ayant 8 ou 9 atomes de carbone sont utilisés.

12. Procédé selon la revendication 11,
**caractérisé en ce que,**
des mélanges d'oléfines en C₈, qui sont fabriqués par oligomérisation sur un lit solide catalytique à base de nickel, sont utilisés.
